# EUROPEAN PATENT SPECIFICATION

(11) **EP 4 069 119 B1**
(45) Date of publication and mention of the grant of the patent: **28.10.2026**
(21) Application number: 20895278.8
(22) Date of filing: 04.12.2020
(51) Int. Cl.: A61B 18/12, A61B 18/04, A61B 18/00, A61B 17/00, A61B 18/14

(54) **DEVICE FOR CALCULATING THE AMOUNT OF ENERGY DELIVERED TO TISSUE DURING AN ELECTROSURGICAL TREATMENT**
VORRICHTUNG ZUM BERECHNEN DER ENERGIEMENGE, DIE WÄHREND EINER ELEKTROCHIRURGISCHEN BEHANDLUNG AN DAS GEWEBE ABGEGEBEN WIRD
DISPOSITIF DE CALCUL DE QUANTITÉ D'ÉNERGIE ADMINISTRÉE AU TISSU PENDANT UN TRAITEMENT ÉLECTRO-CHIRURGICAL

(30) Priority: 07.12.2019 US 201962945142 P
(43) Date of publication of application: 12.10.2022
(73) Proprietor: Apyx Medical Corporation, Clearwater, FL 33760-4004 (US)
(72) Inventor: SHILEV, Nikolay Dimitrov, 1324 Sofia (BG); TOMOV, Viktor Tomov, 1330 Sofia (BG); JONSSON, Fredrik, St. Petersburg, FL 33701 (US); ROMAN, Shawn D., Safety Harbor, FL 34695 (US)
(74) Representative: McDonough, Jonathan
(86) International application number: PCT/US2020/063187
(87) International publication number: WO 2021/113561

(56) References cited:
- WO-A1-2019/147568
- WO-A1-2020/251886
- US-A- 5 827 277
- US-A1- 2012 239 024
- US-A1- 2015 257 817
- US-A1- 2015 320 479
- US-A1- 2017 049 496
- US-A1- 2017 196 617

## Description

### BACKGROUND

### Field.

The present disclosure relates generally to electrosurgery and electrosurgical systems and apparatuses, and more particularly, devices, systems and methods for calculating the amount of energy delivered to tissue during an electrosurgical treatment.

### Description of the Related Art

High frequency electrical energy has been widely used in surgery and is commonly referred to as electrosurgical energy. Tissue is cut and bodily fluids are coagulated using electrosurgical energy.

Electrosurgical instruments generally comprise "monopolar" devices or "bipolar" devices. Monopolar devices comprise an active electrode on the electrosurgical instrument with a return electrode attached to the patient. In monopolar electrosurgery, the electrosurgical energy flows through the active electrode on the instrument through the patient's body to the return electrode. Such monopolar devices are effective in surgical procedures where cutting and coagulation of tissue are required and where stray electrical currents do not pose a substantial risk to the patient.

Bipolar devices comprise an active electrode and a return electrode on the surgical instrument. In a bipolar electrosurgical device, electrosurgical energy flows through the active electrode to the tissue of a patient through a short distance through the tissue to the return electrode. The electrosurgical effects are substantially localized to a small area of tissue that is disposed between the two electrodes on the surgical instrument. Bipolar electrosurgical devices have been found to be useful with surgical procedures where stray electrical currents may pose a hazard to the patient or where other procedural concerns require close proximity of the active and return electrodes. Surgical operations involving bipolar electrosurgery often require methods and procedures that differ substantially from the methods and procedures involving monopolar electrosurgery.

Gas plasma is an ionized gas capable of conducting electrical energy. Plasmas are used in surgical devices to conduct electrosurgical energy to a patient. The plasma conducts the energy by providing a pathway of relatively low electrical resistance. The electrosurgical energy will follow through the plasma to cut, coagulate, desiccate, or fulgurate blood or tissue of the patient. There is no physical contact required between an electrode and the tissue treated.

Electrosurgical systems that do not incorporate a source of regulated gas can ionize the ambient air between the active electrode and the patient. The plasma that is thereby created will conduct the electrosurgical energy to the patient, although the plasma arc will typically appear more spatially dispersed compared with systems that have a regulated flow of ionizable gas.

The amount of energy delivered to patient tissue by the plasma outputted by the electrosurgical system, e.g., by an applicator or handpiece, is not the same as the amount of energy generated and outputted by the electrosurgical generator of the electrosurgical system. Some of the energy outputted by the electrosurgical generator is lost in producing a plasma beam in addition to other inefficiencies. Knowing the amount of energy delivered to patient tissue, and not just the amount of energy outputted by the electrosurgical generator of a system, is useful for producing the desired results in a given treatment. However, currently used electrosurgical systems do not provide a simple and efficient mean by which to accurately measure the energy delivered to patient tissue. Thus, a need exists for devices, systems, and methods that calculate the amount of energy delivered to patient tissue.
US2015/0320479A1 (Cosman et al) discloses high-frequency ablation of tissue in the body using a cooled high-frequency electrode connected to a high frequency generator including a computer graphic control system and an automatic controller for control the signal output from the generator, and adapted to display on a real time graphic display a measured parameter related to the ablation process and visually monitor the variation of the parameter of the signal output that is controlled by the controller during the ablation process. In one example, one or more measured parameters are displayed simultaneously to visually interpret the relation of their variation and values. In one example, the displayed one or more parameters can be taken from the list of measured voltage, current, power, impedance, electrode temperature, and tissue temperature related to the ablation process.
US2017/0049496A1 (Bovie Medical Corporation) discloses systems and methods for discriminating between various gases, e.g., argon and helium, for enhanced safety of medical devices. In one embodiment, a specific gas, helium for example, is expected, and an enabling or "Go" signal is issued when that gas is detected, or an inhibitory or "No-Go" signal when a gas is present that is not identified as helium. Alternately, a control line or input command can be used to select between helium and argon as the expected gas and then issue the appropriate "Go/No-Go" signal, depending on which gas is identified.
US5,827,277 (Edwards, Stuart) discloses a method and apparatus for ablating at least a portion of a nasal concha. The apparatus includes a catheter having a distal portion with a dimension configured for positioning through a nostril of a patient into a nasal meatus adjacent a nasal concha, and an energy delivery device coupled to the catheter distal portion including one or more energy delivering probes extendable from the catheter distal portion a sufficient distance to be inserted into an interior of the nasal concha to deliver ablative energy therein. The distal portion of the apparatus may also include an expandable member, expansion of the expandable member within the nasal meatus immobilizing the distal portion within the nasal meatus.
US2012/239024A1 discloses an electrosurgical generator which includes sensor circuitry configured to measure voltage and current provided by the generator to tissue and a controller configured to measure the time of provision of energy by the controller and to calculate energy provided, the controller further configured to estimate a size of an ablation volume as a function of energy provided by the generator and time and to calculate a growth rate of the ablation volume based on the estimated size.

### SUMMARY OF THE INVENTION

The present invention provides an electrosurgical generator as claimed in claim 1. The methods disclosed herein do not form part of the claimed invention.

### SUMMARY OF THE DISCLOSURE

The present disclosure relates to devices, systems and methods for calculating the amount of energy delivered to tissue during an electrosurgical treatment.

In one aspect, the output power is determined based on a selected generator power setting.

In another aspect, the output power is determined based on sampling output voltage and current of the RF output stage.

In another aspect, the electrosurgical generator further includes an input/output interface that displays the amount of energy delivered to the patient tissue;
wherein, the amount of energy delivered to the patient tissue is displayed in Joules.

In a further aspect, the controller counts the energy delivered to the patient tissue based on the selected power setting and a duration of activation time of the applicator at the selected power setting.

In yet another aspect, the electrosurgical generator further comprises at least one sensor coupled to an output of the RF output stage, the sensor configured to sample voltage and/or current of the RF output of the stage and provide the sampled voltage and/or current to the controller;
wherein the selected generator power setting is determined from the sampled voltage and/or current of the RF output stage.

In one aspect, the electrosurgical generator further incudes at least one sensor that measures impedance at the RF output stage and provides the measured impedance to the controller, wherein the controller determines if the applicator is applying energy to the patient tissue or to ambient air based on the measured impedance and the controller adds the delivered energy to the count only when the applicator is applying energy to the patient tissue.

In another aspect, the electrosurgical generator further includes an input/output interface that enables selection of an energy endpoint for a procedure, wherein the controller causes the power supply to stop supplying electrosurgical energy to the applicator when the count exceeds the energy endpoint.

In one aspect, the controller triggers a notification via the input/output interface when the count exceeds the energy endpoint.

In still a further aspect, the memory stores a predetermined energy endpoint for each of a plurality of procedures;
wherein the input/output interface enables selection of at least one of the plurality of procedures, wherein upon selection of at least one procedure, the controller retrieves a corresponding energy endpoint from the memory.

In another aspect, the at least one energy quantification function is selected based on a type of applicator.

In one aspect, the applicator and at least one receptacle are configured such that at least one energy quantification function is received from the applicator upon coupling the applicator to at least one receptacle.

In a further aspect, the electrosurgical generator further includes an input/output interface that enables storing in memory a total count of energy delivered to a first treatment area of a patient as an energy endpoint, wherein upon selection of a procedure for a contralateral treatment area of the patient, the controller retrieves a stored energy endpoint from the memory.

In one aspect, upon completion of a procedure to a first treatment area of a patient, the controller determines a total amount of energy delivered to the patient tissue and stores the determined total amount of energy in the memory as an energy endpoint for a procedure to a contralateral treatment area of the patient;

the electrosurgical generator further comprising an input/output interface that enables selection of a procedure for the contralateral treatment area, wherein upon selection of the procedure, the controller retrieves the stored energy endpoint from the memory.

According to one aspect of the present disclosure, the electrosurgical generator further includes a flow controller that provides at least one gas to the applicator, wherein the applicator generates plasma from the electrosurgical energy and the at least one gas, the plasma to be delivered to the patient tissue;
wherein the controller counts the energy delivered to the patient tissue based on at least one of a type of the at least one gas, a flow rate of the at least one gas and/or a power setting of the electrosurgical setting.
wherein the counted amount of energy delivered to the patient tissue is displayed in Joules.

In a further aspect, the electrosurgical generator further includes an input/output interface that displays the amount of energy delivered to the patient tissue, wherein the amount of energy delivered to the patient tissue is displayed in Joules per second.

According to another aspect of the present disclosure, a method of performing a medical procedure is provided including applying, via an electrosurgical generator, electrosurgical energy to patient tissue; determining an amount of energy delivered to the patient tissue based on at least one energy quantification function and output power of the electrosurgical generator; comparing the determined amount of energy delivered to an energy endpoint; and stopping application of the electrosurgical energy when the determined amount of energy delivered meets or exceeds the energy endpoint.

In one aspect, the method further includes displaying the amount of energy delivered to patient tissue via an input/output interface of the electrosurgical generator.

In another aspect, the displayed amount of energy delivered is instantaneous energy being delivered in Joules/second.

In a further aspect, the displayed amount of energy delivered is an accumulated count of energy delivered in Joules.

In yet another aspect, the method further includes triggering a notification when the amount of energy delivered meets or exceeds the energy endpoint.

In one aspect, the method further includes storing in a memory a predetermined energy endpoint for each of a plurality of procedures.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is an illustration of an electrosurgical system in accordance with an embodiment of the present disclosure;
FIG. 2A is a front view of an electrosurgical generator of the electrosurgical system of FIG. 1 in accordance with an embodiment of the present disclosure;
FIG. 2B is a block diagram of an electrosurgical generator of the electrosurgical system of FIG. 1 in accordance with an embodiment of the present disclosure;
FIG. 3 is a flowchart illustrating a method for determining an equation for calculating the amount of energy delivered to patient tissue by an applicator of the electrosurgical system of FIG. 1 in accordance with an embodiment of the present disclosure;
FIG. 4 is a flowchart illustrating a method for counting the amount of energy delivered to patient tissue by the applicator of the electrosurgical system of FIG. 1 in accordance with an embodiment of the present disclosure;
FIG. 5 illustrate exemplary results of a method for determining an equation for calculating the amount of energy delivered to patient tissue by an applicator of an electrosurgical system in accordance with an embodiment of the present disclosure;
FIG. 6 is a graph used for determining a Joule counter equation in accordance with an embodiment of the present disclosure; and
FIG. 7 is a flowchart illustrating a method for applying electrosurgical energy to different portions of a patient using an electrosurgical system in accordance with an embodiment of the present disclosure.

It should be understood that the drawings are for purposes of illustrating the concepts of the disclosure and are not necessarily the only possible configuration for illustrating the disclosure.

### DETAILED DESCRIPTION

Preferred embodiments of the present disclosure will be described hereinbelow with reference to the accompanying drawings. In the following description, well-known functions or constructions are not described in detail to avoid obscuring the present disclosure in unnecessary detail. In the drawings and in the description which follow, the term "proximal", as is traditional, will refer to the end of the device, e.g., instrument, apparatus, applicator, handpiece, forceps, etc., which is closer to the user, while the term "distal" will refer to the end which is further from the user. Herein, the phrase "coupled" is defined to mean directly connected to or indirectly connected with through one or more intermediate components. Such intermediate components may include both hardware and software based components.

It will be appreciated by those skilled in the art that the block diagrams presented herein represent conceptual views of illustrative circuitry embodying the principles of the disclosure. Similarly, it will be appreciated that any flow charts, flow diagrams, state transition diagrams, pseudo-code, and the like represent various processes which may be substantially represented in computer readable media and so executed by a computer or processor, whether or not such computer or processor is explicitly shown.

The present disclosure relates to devices, systems and methods for calculating the amount of energy delivered to tissue during an electrosurgical treatment.

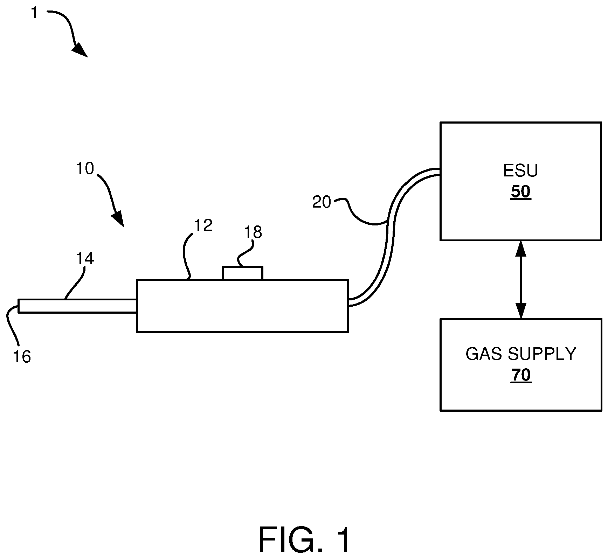

Referring to FIG. 1, an electrosurgical system 1 is shown in accordance with the present disclosure. System 1 includes an applicator or handpiece 10 and an electrosurgical generator unit (ESU) 50. In some embodiments, system 1 further includes a gas supply 70.

Applicator 10 is configured to receive electrosurgical energy from ESU 50 via a cable 20. Applicator 10 is further configured to receive an inert gas from a gas source 70. In some embodiments, the inert gas is received from a gas supply 70 and provided from ESU 50 to applicator 10 via cable 20. It is to be appreciated that gas supply 70 may be internal to ESU 50 or external to ESU 50. In other embodiments, applicator 10 receives the inert gas directly from gas supply 70. Applicator 10 includes a handle housing 12 having a button 18 and a shaft 14 having a distal tip 16. When button 18 is pressed, electrosurgical energy is delivered to applicator 10 by ESU 50 and inert gas is delivered to applicator 10 by the gas source 70. The electrosurgical energy is used to energize an electrode disposed in shaft 14. When the inert gas is passed over the energized electrode, a plasma is generated and emitted from tip 16 to patient tissue, which allows for conduction of the radio frequency (RF) energy from the electrode to the patient in the form of a precise plasma beam. In one embodiment, helium is used as the inert gas because helium can be converted to a plasma with very little energy, however, other inert gases, such as argon, are considered within the scope of the present disclosure. Additionally, mixtures of inert gases may be utilized to generate a plasma. Exemplary applicators are shown and described in commonly-owned U.S. Patent No. 9,060,765.

It is to be appreciated that, in some embodiments, applicator 10 may be configured to apply or deliver energy to patient tissue in ways or forms other than plasma. For example, applicator 10 may deliver RF energy to patient tissue via direct contact of the electrode to patient tissue. In some embodiments, the electrode may be retractable within shaft 14 to enable the electrode to be extended and used to directly contact patient tissue to deliver RF energy or retract to deliver RF energy via plasma. In other embodiments, the electrode may be configured as a probe or heating element (e.g., heated by applying current received from ESU 50 to the heating element) and heat energy may be applied directly to patient tissue by the heat element.

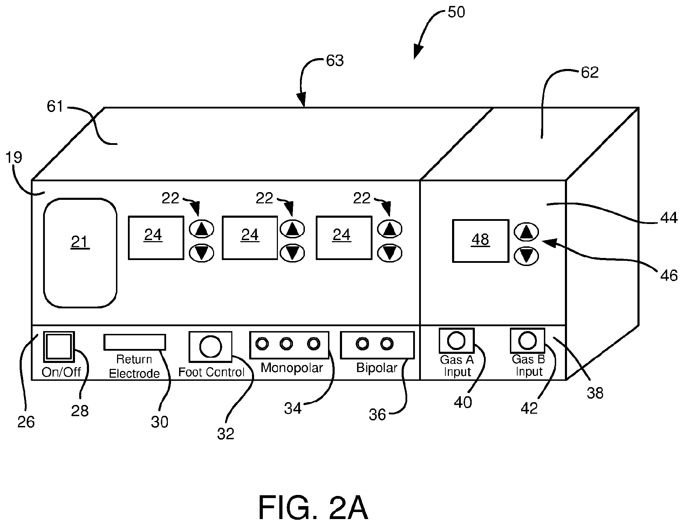

Referring to FIG. 2A, a front view of ESU 50 is shown in accordance with an embodiment of the present disclosure. In one embodiment, the ESU 50 includes a high frequency electrosurgical generator 61 and gas flow controller 62 contained in a single housing 63. The ESU 50 includes a front panel face 19 which includes an input/output section 21, e.g. a touchscreen, for entering commands/data into the ESU 50 and for displaying data. The front panel 19 may further include various level controls 22 with corresponding indicators 24. Additionally, the ESU 50 includes a receptacle section 26 which may include an On/Off switch 28, a return electrode receptacle 30, a monopolar foot-switching receptacle 32, monopolar hand-switching receptacle 34 and a bipolar hand-switching receptacle 36. The gas flow controller 62 includes a gas receptacle portion 38 which may further include a Gas A input receptacle 40 and a Gas B input receptacle 42. The gas flow controller 62 may further include a user interface portion 44 including selector switch or input 46 and a display 48. The selector switch or input 46 enables selection of the type of gas being input, selection of a mixture of gases being input, a composition and/or percentages of a mixture of gases being input, a flow rate of a gas being applied to a handpiece or applicator, etc. It is to be appreciated that although FIG. 2A shows the high frequency electrosurgical generator 61 and gas flow controller 62 housed in a single housing 63, gas flow controller 62 may be provided as a separate, external device which interfaces with the ESU 50, via a wired and/or wireless interface.

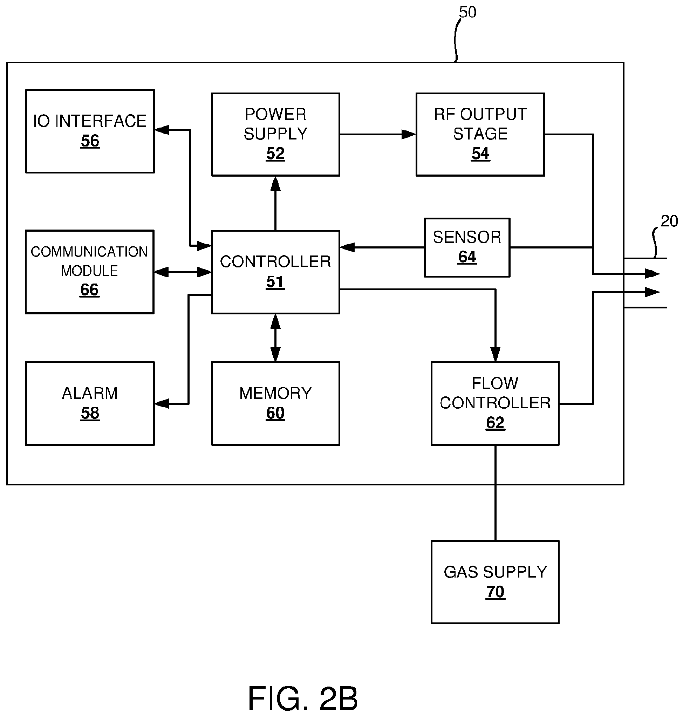

Referring to FIG. 2B, a block diagram of ESU 50 is shown in accordance with an embodiment of the present disclosure. ESU 50 includes controller or processor 51, power supply 52, radio frequency (RF) output stage 54, I/O interface 56, alarm 58, memory 60, flow controller 62, sensor 64, and a communication module 66. Controller 51 is configured to control power supply 52 to supply electrosurgical energy being output from RF output stage 54 via at least one conductor extending through cable 20 to the applicator 10. It is to be appreciated that cable 20 may be coupled to ESU 50 via monopolar hand-switching receptacle 34 or bipolar hand-switching receptacle 36. I/O interface 56 is configured to receive user input (e.g., via one or more buttons 22, 46, touchscreens 21, etc., disposed on the housing of ESU 50) to be provided to the controller 51 and output information (e.g., data to indicators 24, graphical user interfaces to touchscreen 21 , etc.) received from controller 51. Audible alarm 58 is controllable via controller 51 to alert an operator to various conditions or events.

Flow controller 62 is configured for controlling the flow of gas received from supply 70 to the applicator 10. The flow controller 62 is coupled to the controller 51 and receives control signals from the controller 51 based on user input via I/O interface 56, selector switch or input 46 or based on an algorithm or software function stored in memory 60. Additionally, the flow controller 62 may include appropriate sensors to determine a type of gas being input to receptacles 40, 42. Furthermore, the flow controller 62 may use the inputted gases to create a mixture of gases to be provided to the applicator. Although in the embodiment shown in FIG. 2B, the flow controller 62 is disposed in the ESU 50, the flow controller 62 can be located external to the ESU 50 and disposed, for example, in a separate housing, in the applicator 10, etc.

Communication module 66 of ESU 50 is configured to communicate with other devices (e.g., client devices, servers, etc.) via a communication link (e.g., wired or wireless) to send and receive data and communications. Although in the embodiment shown in FIG. 2B, an operator is alerted to various conditions via an audible alarm 58, in other embodiments, controller 51 may use communication module 66 to send notifications to at least one other device via the communication link (e.g., wired or wireless), where the communications are associated with the various conditions or events. The communication module 66 may be a modem, network interface card (NIC), wireless transceiver, etc. The communication module 66 will perform its functionality by hardwired and/or wireless connectivity. The hardwire connection may include but is not limited to hard wire cabling e.g., parallel or serial cables, RS232, RS485, USB cable, Firewire (1394 connectivity) cables, Ethernet, and the appropriate communication port configuration disposed on a surface of housing 63. The wireless connection may operate under any of the various wireless protocols including but not limited to Bluetooth^{™} interconnectivity, infrared connectivity, radio transmission connectivity including computer digital signal broadcasting and reception commonly referred to as Wi-Fi or 802.11.X (where x denotes the type of transmission), satellite transmission or any other type of communication protocols, communication architecture or systems currently existing or to be developed for wirelessly transmitting data including spread spectrum 900 MHz, or other frequencies, Zigbee, and/or any mesh enabled wireless communication.

In one embodiment, sensor 64 of ESU 50 is coupled to the output of RF output stage 54. Sensor 64 is configured to sample the voltage and/or current (or any other electrical properties) of the output of stage 54 and provide the sample voltage and/or current to controller 51. Controller 51 may use the information to determine one or more properties associated with the power provided by ESU 50 to applicator 10. In one embodiment, sensor 64 may include at least one voltage sensor for sensing output voltage and at least one current sensor for sensing output current. Optionally, sensor 64 may include at least one analog-to-digital converter for converting the sensed signal to a digital signal to be input to controller 51; or alternatively, at least one analog-to-digital converter may be provided on controller 51.

In one embodiment, controller 51 is configured to determine the amount of energy delivered, e.g., in Joules, to patient tissue by applicator 10 during a treatment. Controller 51 executes an energy quantification algorithm or function (e.g., stored in memory 60 of ESU 50) that enables the controller 51 of ESU 50 to determine the amount of energy delivered to patient tissue by applicator 10 over a period of time. The algorithm or function makes use of an equation or a look-up table for determining the energy delivered to the tissue. As will be described below, in one embodiment, the equation is based on the results of calorimeter testing.

It is to be appreciated that the functions of the ESU 50 shown in FIGS. 1 and 2A-B may be provided through the use of dedicated hardware as well as hardware capable of executing software in association with appropriate software. In one embodiment, some or all of the functions of controller 51 may be performed by at least one processor, such as a computer or an electronic data processor, digital signal processor or embedded micro-controller, field programmable gate array (FPGA), in accordance with code, such as computer program code, software, firmware, register transfer logic and/or integrated circuits that are coded to perform such functions, unless indicated otherwise. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, read only memory (ROM) for storing software and/or firmware, random access memory (RAM), and nonvolatile storage.

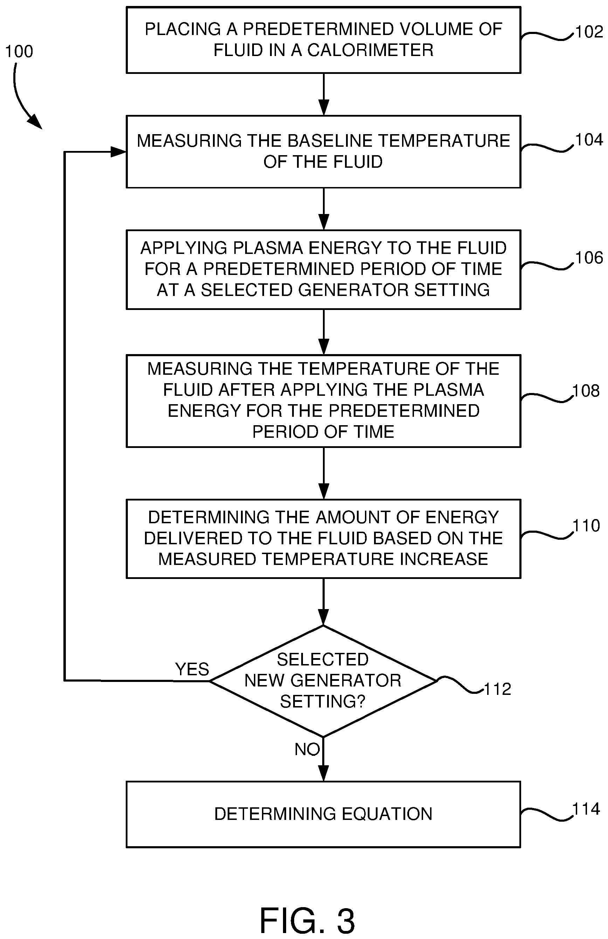

Referring to FIG. 3, a method 100 for determining an equation for calculating the amount of energy delivered to patient tissue by applicator 10 is shown in accordance with an embodiment of the present disclosure.

In step 102, a predetermined volume of fluid (e.g., saline) is placed in a calorimeter. In step 104, the calorimeter is used to measure the baseline temperature of the volume of fluid. In step 106, a first generator setting is selected, e.g., via touchscreen 21 or an appropriate level control 22, and applicator 10 is used to apply plasma energy (or other types of energy, e.g., RF energy via direct contact of the electrode to the volume of fluid, heat energy via direct contact of the electrode or heating element to the volume of fluid, etc.) to the volume of fluid for a predetermined period or length of time. It is to be appreciated that, so long as the distal tip 16 is held within a sufficient distance from the surface of the volume of fluid (or to patient tissue) to enable a plasma arc to occur between the distal tip 16 and the surface of the fluid (or to patient tissue), the tip 16 may be held at any distance within this sufficient distance without causing more heat to be delivered to the fluid (or to patient tissue). As such, once within the sufficient distance, tip 16 may be placed closer or further from a fluid surface or patient tissue without changing the amount of heat delivered. It is to be appreciated that the generator setting of ESU 50 represents an amount of power delivered by ESU 50 (e.g., from RF output stage 54) to applicator 10. In one embodiment, the power setting is expressed as a percentage of the max power deliverable by ESU 50 to applicator 10. For example, in one embodiment, the max output power deliverable from the ESU 50 to applicator 10 may be 40 Watts (W). Thus, setting the ESU 50 to a setting of 20% will result in an output of 20% of 40W (i.e., 8W).

In step 108, the calorimeter is used to measure the temperature of the volume of fluid after applying the plasma energy for the predetermined period of time. In step 110, the amount of energy that was delivered to the volume of fluid by applicator 10 is calculated by calculating the amount of energy needed to increase the temperature of the known volume of fluid from the baseline temperature measured in step 104 to the temperature measured in step 108 over the predetermined period of time. The data from steps 104-110 are recorded in an energy delivery chart or table (e.g., stored in a memory, such as memory 60). In one embodiment, the temperature measured in step 108 is inputted to the ESU 50, for example, via input/output section 21. The amount of energy needed to increase the temperature of the known volume of fluid from the baseline temperature measured in step 104 to the temperature measured in step 108 may then be calculated by an algorithm or function stored in memory 60 and executed by controller 51. In this manner, the energy delivery chart or table may be generated by the controller 51 and stored in memory 60 for later use.

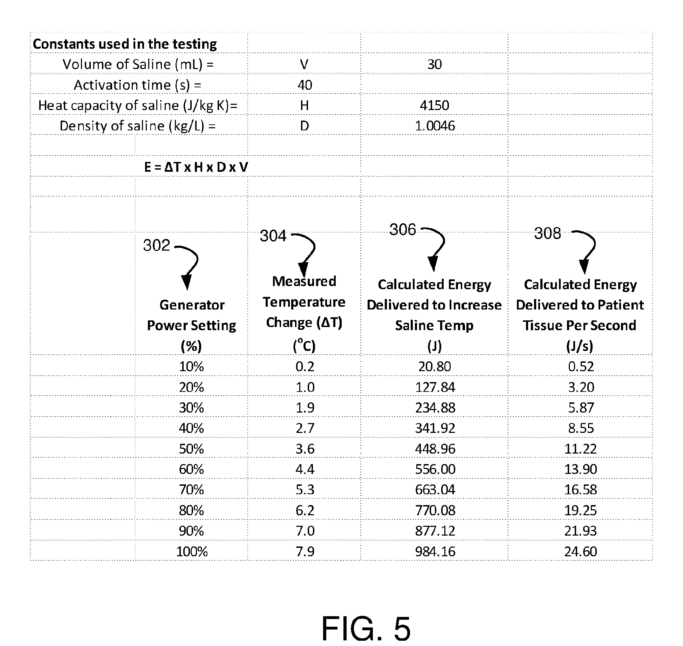

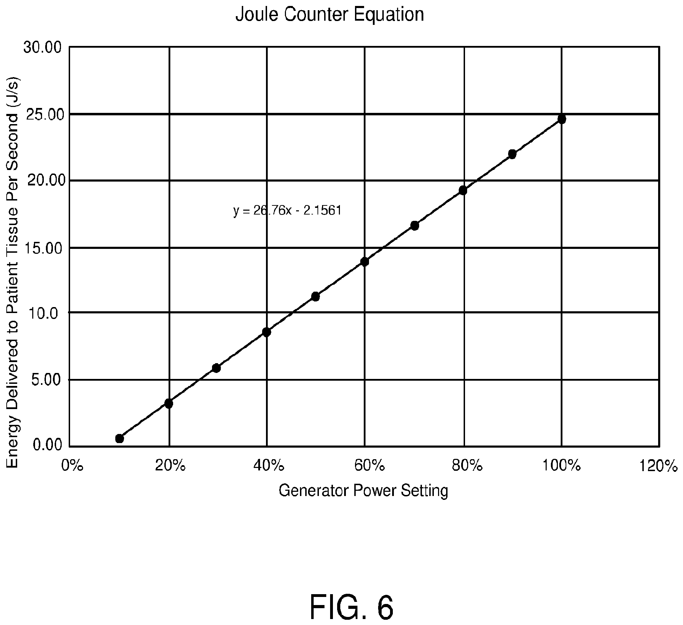

In step 112, a new generator setting is selected via input/output section 21 or an appropriate level control 22 (e.g., in one embodiment incrementing the percentage of power delivered up by a predetermined increment amount), and steps 104-112 are performed until the maximum power setting for ESU 50 is reached. In this way, the energy delivery chart includes the amount of energy being delivered for the predetermined period of time over multiple different generator settings of ESU 50. In step 114, based on the data collected in steps 104-112, the energy delivery (or Joule counter equation) chart is generated and an equation, e.g., an energy quantification function, for calculating the amount of energy delivered to patient tissue by applicator 10 is determined based, at least in part, on the energy delivery chart, the details of which will be described in detail below in relation to FIGS. 5 and 6.

It is to be appreciated that method 100 may be used to determine an equation, e.g., an energy quantification function, for calculating the amount of energy delivered to patient tissue via any energy delivery means (e.g., RF energy delivery via a plasma arc between tip 16 and a fluid or patient tissue, RF energy delivery via direct contact of an electrode of applicator 10 and a fluid or patient tissue, heat energy deliver via direct contact of a heat element of applicator 10 and a fluid or patient tissue).

In one embodiment, the equation determined in step 114 is as follows: $Y = AX + B \left(Equation 1\right) \left(for x>=10 and <=100\right)$ (1)

In the equation 1 above, Y is equal to the energy delivered to patient tissue by applicator 10 per second, X is the generator power setting (e.g., a percentage of the maximum power deliverable by ESU 50 to applicator 10), and A and B are constants determined based on the energy delivery chart constructed in step 114 of method 100. It is to be appreciated that constants A and B will vary depending on the electrical properties of ESU 50 and applicator 10. Thus, where ESU 50 is used with applicators 10 having different electrical properties, memory 60 of ESU 50 may store the different values of constants A and B that are associated to each different applicator 10 that can be used with ESU 50. Alternatively, a connector of each applicator may include a memory which stores the constants A and B and transfers the constants to controller 51 upon coupling the application to the ESU 50.

Exemplary results of the method of FIG. 3 are illustrated in FIGS. 5 and 6. The results of steps 104 through 112 of method 100 are shown in FIG. 5. For each generator power setting 302, a measured temperature change (ΔT) 304 of the fluid, e.g., saline, is recorded. The energy delivered to increase the saline temperature 306 is then calculated for each generator power setting 302 using the following formula: ${E}_{S} = ΔT \times H \times D \times V$ (2) Where ΔT is the measured temperature change 304, H is the heat capacity of saline (J/kg K) = 4150, D is the density of saline (kg/L)= 1.0046 and V is the volume of saline (mL) = 30. The calculated energy delivered to increase the saline temperature (Es) 306 is then used to calculate energy delivered to Patient Tissue per second 308 using the following formula: ${E}_{p} = {E}_{S} / activation time$ (3) Where the activation time is 40 seconds. The energy delivered to patient tissue E_{P} 308 data is then plotted for each generator power setting 302 employed, as shown in FIG. 6. A linear line of best fit is applied to the data. The slope of the line of best fit = A and the y-intercept of the line of best fit = B. For the data shown in FIG. 5, A = 26.76 and B = - 2.1561.Using equation 1 with the determined constants A and B, the energy delivered to tissue E_{P} 308 may be determined for a given generator power setting X.

It is to be appreciated that other variables or factors may be considered when determining the energy quantification function or energy delivery chart of the present disclosure. In one embodiment, the method 300 may be performed using different types of inert gas and an energy quantification function may then be generated and stored for each type of gas. In another embodiment, the method 300 may be performed using different mixtures of gases and an energy quantification function may then be generated and stored for each mixture of gases. For example, a density of a mixture of gases may be determined based on the composition of the mixture and the density of each gas. The density of the mixture of gases may be then be employed to select the proper energy quantification function or energy delivery table. It is to be appreciated that the density of the gas mixture may be selected via input section 21, selector input 46 or may be determined automatically by the ESU 50, for example, flow controller 62 incorporating the proper sensors. In another embodiment, the method 300 may be performed using different flow rates for a predetermined gas and an energy quantification function may then be generated and stored for each flow rate of the predetermined gas. It is to be appreciated that a single variable or various combinations of variables may be used to generate and select an appropriate energy quantification function or energy delivery table. For example, upon selection of a type of gas and flow rate, the controller 51 may select a corresponding energy quantification function or energy delivery table. In another example, upon selection of a mixture of gases and flow rate, the controller 51 may select the corresponding energy quantification function or energy delivery table.

In one embodiment, controller or processor 51 of ESU 50 is configured to determine (e.g., based on user input received via interface 56 or automatically by communicating with a memory or processor of applicator 10) the type of applicator 10 coupled to ESU 50 and to use the appropriate constants A, B in equation 1. For example, in one embodiment, A = 26.76 and B = -2.1561. In this example, if the generator setting is set to 50% of the max power, the applicator 10, while active and receiving power from ESU 50, will deliver 11.22 Joules per second to patient tissue as determined as follows: $Y = \left(26.76\right) * \left(0.50\right) - 2.1561 = 11.22 .$

In one embodiment of the present disclosure, sensor 64 is configured to sample the output of RF output stage 54 for a voltage and current reading. The sampled voltage and/or current is provided to controller 51, where controller 51 is configured to determine the amount of power being outputted by the RF output stage 54 and provided to applicator 10 based on the sampled voltage and current. The amount of power being provided to applicator 10 may be used by controller 51 to determine the current generator setting X in real time to increase the accuracy of the energy delivered to the patient calculations using the equation 1 described above. For example, the controller 51 may determine the amount of power being delivered to the applicator 10 based on actual voltage and current reading is different that the power setting that was input to the generator, i.e., the determined power is 55% while the inputted power setting is 50%. The controller 51 may use the determined power percentage to more accurately determine the energy delivered to patient tissue.

In another embodiment, the samplings from sensor 64 are used by controller 51 to calculate the amount of power delivered by applicator 10 to patient tissue. For example, the samplings (e.g., voltage and/or current) and any associated calculation and/or electrical properties (e.g., impedance) of the output of stage 54 may be mapped by controller 51 to various temperatures of a sample fluid (in the manner described above with respect to method 100) to determine energy delivered to the patient tissue based on the calculated power at the RF output stage 54. Thereafter, controller 51 is configured to sample the output of stage 54 during a procedure and, based on the saved mapping and the sampling of the output of stage 54, controller 51 is configured to determine the amount of energy being delivered to patent tissue by applicator 51 during the procedure. For example, a look-up table may be programmed into the generator based on the above following equation: $Y = AZ + B$ (4) Where Y is equal to the energy delivered to patient tissue by applicator 10 per second, Z is the calculated output power, and A and B are constants determined based on the energy delivery chart constructed in step 114 of method 100. In this example, A=0.669 and B= -2.1561 (for x>=4 and <=40). The controller 51 samples the output stage 54 and determines the power output (Z). On the look-up table, the power output (Z) corresponds to the power being delivered to the patient (Y) based on the equation. Knowing Y(J/s) and the amount of activation time. The generator may determine the amount of energy delivered to the patient.

In one embodiment of the present disclosure, the equation 1 determined in step 114 and described above is stored in memory 60 of ESU 50 and executed by controller 51 during an electrosurgical procedure to determine the amount of energy applied to patient tissue. In this embodiment, the controller 51 uses at least two pieces of data to calculate or count the energy delivered to the patient tissue: (1) the generator power setting (i.e., X in equation 1); and (2) the length or duration of activation time at the power setting. Controller 51 is configured to continuously track the current power setting of ESU 50 (e.g., using data from sensor 64 and/or tracking the user selections received from I/O interface 56) and the activation time at the current power setting to determine or count the amount of energy delivered to patient tissue. It is to be appreciated that as applicator 10 is turned on and off to apply plasma to tissue and discontinue the application of plasma to tissue and the power setting of ESU 50 is changed, controller 51 is continuously calculating or counting the amount of power delivered to patient tissue using equation 1, described above.

In one embodiment, controller 51 may be configured to determine if applicator 10 is actually applying energy to the patient tissue and not to the ambient air or another target that is not the patient tissue. In this embodiment, controller 51 uses the samplings from sensor 64, e.g., voltage and current readings or samples, to determine the impedance or change in impedance at the output of RF output stage 54. Based on the impedance or change in impedance, controller 51 is configured to determine if the energy outputted by applicator 10 is being applied to patient tissue. For example, controller 51 may determine that if the impedance is at or above a predetermined level or value, then the energy is being applied to patient tissue. As another example, controller 51 may determine that if the impedance has changed by a predetermined level or value, then the energy is being applied to patient tissue. In any case, controller 51 is configured to count the energy applied to the patient tissue using equation 1 only while controller 51 determines that the energy is being applied to patient tissue by applicator 10 and not to the ambient are or a target other than the patient tissue.

In one embodiment, the energy delivered to the patient tissue that is calculated by controller 51 is outputted for display in Joules by controller 51 to a display of ESU 50 via I/O interface 56, e.g., displayed on touchscreen 21. It is to be appreciated that the input/output section 21 may display instantaneous energy being delivered in Joules/second, the accumulated count of energy delivered in Joules or both simultaneously. I/O interface 56 is configured to receive user input (e.g., via one or more buttons 22, a touch screen 21, etc., of ESU 50) to enable a user to set an energy counter of controller 51 to zero and also to set an energy endpoint.

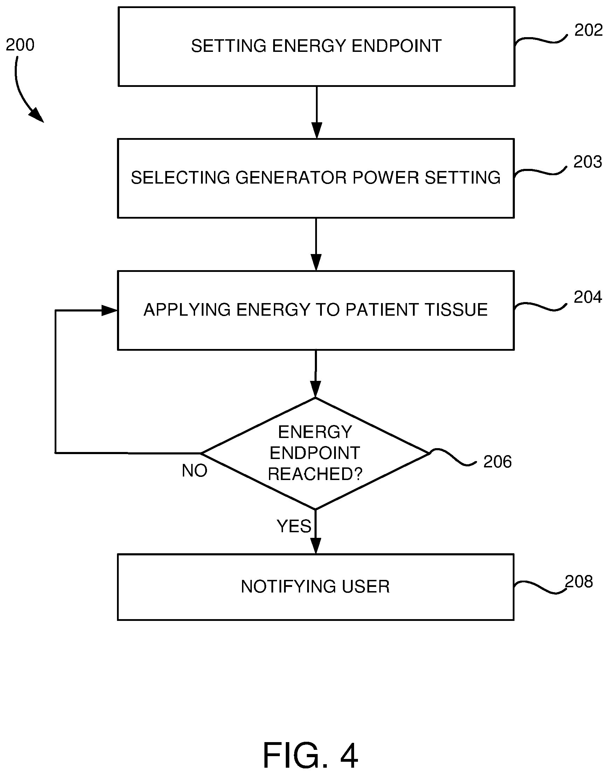

Referring to FIG. 4, a method 200 for counting the amount of energy delivered to patient tissue by applicator 10 is shown in accordance with an embodiment of the present disclosure. In step 202, via user input received by I/O interface 56, an energy endpoint is set. It is to be appreciated that the energy endpoint may be selected indirectly by selecting a type of procedure, e.g., tissue tightening, and/or a type of procedure for a specific anatomical location, e.g., dermal resurfacing of a cheek. Optionally, a user input may reset the energy or Joule counter to zero before a procedure begins or before starting treatment of a new anatomical location. In step 203, a generator power setting is selected. It is to be appreciated that the generator power setting may be selected manually by an operator of the generator or may be automatically selected based upon a selected type of procedure.

In step 204, applicator 10 is used to apply plasma (or another type of) energy to patient tissue. In step 206, controller 51 is configured to monitor and calculate the amount of energy delivered to patient tissue by applicator 10, based on the selected generator power setting. In one embodiment, the accumulated amount of energy delivered to patient tissue is displayed via input/output section 21 and constantly updated throughout the procedure while the applicator 10 is activated. In another embodiment, the input/output section 21 may display the instantaneous energy being delivered in Joules/second, while also displaying the accumulated count of energy delivered in Joules. In step 206, the accumulated amount of energy delivered is compared to the energy endpoint and, if the energy endpoint is reached, controller 51 notifies the user (e.g., by triggering audible alarm 58, displaying the total amount of Joules delivered on input/output section 21, triggering a blinking indicator on the display of ESU 50, and/or sending a notification to another device or an external device via a communication module) that the energy endpoint has been reached, such that the user stops applying plasma to the patient tissue. In some embodiments, when controller 51 determines that the energy endpoint has been reached, controller 51 automatically causes power supply 52 to stop supplying power to applicator 10, such that additional plasma energy cannot be provided to the patient tissue.

It is to be appreciated that equation 1 described above and method 200 may be used in any type of procedure, electrosurgical or otherwise, where energy is delivered to patient tissue, e.g., via plasma, RF energy via direct contact of an electrode of applicator 10 to patient tissue, and/or heat energy via direct contact of a heat element of applicator 10 to patient tissue. Some procedures that equation 1 and method 200 may be used to calculate the energy delivered to patient tissue may include, but are not limited to, tissue tightening and wrinkle reduction procedures.

In one embodiment, the capability of controller 51 to determine the amount of energy that is delivered by applicator 10 to patient tissue is used to determine the optimal amount of applied energy needed to be applied for a given procedure performed on a given region of a body part. Furthermore, once the optimal energies are determined for various procedures, the energy for each procedure can be stored in memory 60 of ESU 50. Once the energies/procedures are stored, a user may select a stored procedure via I/O section 21 which transmits the selection to controller 51 via I/O interface 56, and controller 51 will retrieve the corresponding energy required for the selected procedure and perform the method 200 described above using the retrieved energy from memory 60 as the energy endpoint. In this way, each time a given procedure is performed, the optimal amount of energy is delivered to the patient tissue, thus ensuring consistent results. It is to be appreciated that the generator power setting for a selected procedure may be manually entered by an operator or, alternately, the generator power setting may be stored with the energy endpoint for a given procedure.

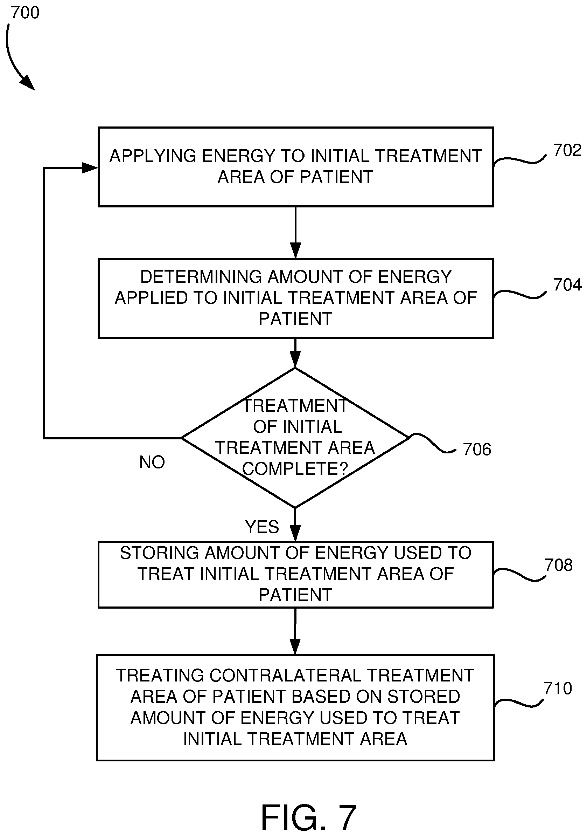

Referring to FIG. 7, a method 700 is provided for with ensuring consistent treatment of different body areas. In step 702, the given or predetermined initial treatment area of the patient is treated by applying electrosurgical energy to patient tissue. During the procedure, an amount of energy, e.g., in Joules, delivered to the predetermined initial treatment area is determined, in step 704, as described above. In step 706, it is determined if the treatment is complete. If the treatment procedure is not complete in step 706, the method may revert to step 702 and electrosurgical energy may continue to be applied to the initial treatment area. Otherwise, if the procedure is complete, the generator, or controller 51, may store or record the amount of energy delivered to the initial treatment area in memory 60, in step 708, to use the amount of energy delivered as the set point or energy endpoint for a contralateral treatment area. In step 710, a contralateral treatment area of the patient is treated using the same amount of energy as applied to the initial treatment area to ensure consistent (balanced) treatment on both sides of the body.

As an example, a user may perform a tissue tightening procedure using applicator 10 and ESU 50 to reduce skin laxity under each of the arms of a patient. To ensure uniform treatment of both arms of the patient, the user may observe the amount of energy delivered to the right arm of the patient that is calculated by controller 51 and document the energy delivered. Alternately, the controller 51 may store the amount of energy delivered in memory 60, where the amount of energy delivered may be associated with the type of procedure/treatment and/or the particular area of the patient and may further be stored as the energy setpoint for a contralateral area. Then, the user may set the documented or stored energy that was applied to the right arm as the energy endpoint via user input to I/O interface 56 before performing the tissue tightening procedure on the left arm. It is to be appreciated that the user may also select the energy endpoint that was stored for the contralateral area via the I/O interface 56. In this way, controller 51 will perform method 200 described above to ensure that the set energy endpoint is not exceeded and the same amount of energy that was applied to the right arm is applied to the left arm. The energy endpoint may be stored in memory 60 and used in future skin tightening procedures on arms.

As another example, a user may perform a dermal resurfacing procedure using applicator 10 and ESU 50 to reduce facial wrinkles. The user may document (via observing the calculations of controller 51) the amount of energy applied by applicator 10 to the right cheek during the resurfacing procedure. Alternately, the controller 51 may store the amount of energy delivered in memory 60, where the amount of energy delivered may be associated with the type of procedure/treatment and/or the particular area of the patient and may further be stored as the energy setpoint for a contralateral area, i.e., the left cheek. Then, the user may set the documented energy as the energy endpoint before performed the resurfacing procedure on the left cheek. It is to be appreciated that the user may also select the energy endpoint that was stored for the contralateral treatment area, i.e., the left cheek, via the I/O interface 56, e.g., touchscreen 21. When the skin resurfacing procedure is performed on the left cheek, controller 51 will perform method 200 described above to ensure that the set energy endpoint is not exceeded and the same amount of energy applied to the right cheek is applied to the left cheek. The energy endpoint may be stored in memory 60 and used in further skin resurfacing procedures.

As data from various procedures is gathered and stored in memory 60, memory 60 will include data for how much energy is needed to perform various procedures (e.g., skin tightening procedures) at various body regions. This data can be used by controller 51 to prevent over-treatment or under-treatment of a body region. For example, if it is determined that 10J of energy must be applied to one region of the body (e.g., a quadrant of the abdomen), a user can select the region of the body via input to I/O interface 56, and the required energy (i.e., 10J) will be retrieved from memory 60 and used by controller 51 as the energy set point to ensure no more than 10J is delivered to patient tissue during the procedure.

It is to be appreciated that the data for performing various procedures may be gathered in several ways. In one embodiment, data is gathered by controller 51 and stored in memory 60 for each procedure performed using ESU 50 and applicator 10. The data accumulated or gathered by each ESU 50 may be extracted either manually (e.g., by a user connecting a device such as a universal serial bus (USB) or other type of device and extracting the data) or automatically (e.g., where controller 51 sends or pushes the data to an external device, such as a server via communication module 66) and provided to a server. Data for procedures may also be gathered and stored by the server via a data registry where users upload the data to the server. The data may be generated from the findings of a clinical trial or the data may be generated from procedures performed by physicians or other professionals at various facilities. In any case, the data on the server may be accessible for use by each ESU 50 via communication module 66 to be used in a procedure. The data on the server may be analyzed to determine an optimal data set that is smaller than the total data set on the server. The optimal data set may be stored in memory 60 and used by controller 51 to perform a procedure according to the data.

lt is to be appreciated that the various features shown and described are interchangeable, that is a feature shown in one embodiment may be incorporated into another embodiment.

Furthermore, although the foregoing text sets forth a detailed description of numerous embodiments, it should be understood that the legal scope of the invention is defined by the words of the claims set forth at the end of this patent. The detailed description is to be construed as exemplary only and does not describe every possible embodiment, as describing every possible embodiment would be impractical, if not impossible. One could implement numerous alternate embodiments, using either current technology or technology developed after the filing date of this patent, which would still fall within the scope of the claims.

It should also be understood that, unless a term is expressly defined in this patent using the sentence "As used herein, the term '_' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning.

## Claims

1. An electrosurgical generator (50, 61) comprising:
a power supply (52) that supplies electrosurgical energy to an applicator (10) via a radio frequency (RF) output stage (54);
a memory (60) that stores at least one energy quantification function that determines an amount of energy delivered to patient tissue by the applicator (10), the at least one energy quantification function associates actual energy delivered to a surface of tissue to the output power of the RF output stage (54); and
a controller (51) that determines the amount of energy delivered to the patient tissue based on the energy quantification function and output power of the RF output stage (54).

2. The electrosurgical generator (50, 61) as in claim 1, wherein the controller is configured to determine the output power based on a selected generator power setting.

3. The electrosurgical generator (50, 61) as in claim 1, wherein the controller is configured to determine the output power based on sampling output voltage and current of the RF output stage (54).

4. The electrosurgical generator (50, 61) as in claim 1, further comprising an input/output interface (56) configured to display the amount of energy delivered to the patient tissue;
wherein the amount of energy delivered to the patient tissue is displayed in Joules.

5. The electrosurgical generator (50, 61) as in claim 2, wherein the controller (51) **is** configured to count the energy delivered to the patient tissue based on the selected power setting and a duration of activation time of the applicator (10) at the selected power setting.

6. The electrosurgical generator (50, 61) as in claim 2 further comprising at least one sensor (64) coupled to an output of the RF output stage (54), the sensor (64) configured to sample voltage and/or current of the RF output stage (54) and provide the sampled voltage and/or current to the controller (51).

7. The electrosurgical generator (50, 61) as in claim 5, further comprising at least one sensor (64) that measures impedance at the RF output stage (54) and provides the measured impedance to the controller (51), wherein the controller (51) determines if the applicator (10) is applying energy to the patient tissue or to ambient air based on the measured impedance and the controller (51) adds the delivered energy to the count only when the applicator (10) is applying energy to the patient tissue.

8. The electrosurgical generator (50, 61) as in claim 1, further comprising an input/output interface (56) that enables selection of an energy endpoint for a procedure, wherein the controller (51) is configured to cause the power supply (52) to stop supplying electrosurgical energy to the applicator (10) when the count exceeds the energy endpoint.

9. The electrosurgical generator (50, 61) as in claim 8, wherein the controller (51) **is** configured to trigger a notification via the input/output interface (56) when the count exceeds the energy endpoint.

10. The electrosurgical generator (50, 61) as in claim 8, wherein the memory (60) stores a predetermined energy endpoint for each of a plurality of procedures;
wherein the input/output interface (56) enables selection of at least one of the plurality of procedures, wherein upon selection of at least one procedure, the controller (51) retrieves a corresponding energy endpoint from the memory (60).

11. The electrosurgical generator (50, 61) as in claim 1, wherein the controller is configured to select the at least one energy quantification function based on a type of applicator (10).

12. The electrosurgical generator (50, 61) as in claim 1, wherein the applicator (10) and at least one receptacle (30, 32, 34, 36, 40) are configured such that at least one energy quantification function is received from the applicator (10) upon coupling the applicator (10) to at least one receptacle (30, 32, 34, 36, 40).

13. The electrosurgical generator (50, 61) as in claim 5, further comprising an input/output interface (56) that enables storing in memory a total count of energy delivered to a first treatment area of a patient as an energy endpoint, wherein upon selection of a procedure for a contralateral treatment area of the patient, the controller (51) retrieves a stored energy endpoint from the memory (60).

14. The electrosurgical generator (50, 61) as in claim 1, wherein upon completion of a procedure to a first treatment area of a patient, the controller (51) determines a total amount of energy delivered to the patient tissue and stores the determined total amount of energy in the memory (60) as an energy endpoint for a procedure to a contralateral treatment area of the patient;
the electrosurgical generator (50, 61) further comprising an input/output interface (56) that enables selection of a procedure for the contralateral treatment area, wherein upon selection of the procedure, the controller (51) retrieves the stored energy endpoint from the memory (60).

15. The electrosurgical generator (50, 61) as in claim 1, further comprising a flow controller (62) configured to provide at least one gas to the applicator (10), wherein the applicator (10) **is** configured to generate plasma from the electrosurgical energy and the at least one gas, the plasma to be delivered to the patient tissue;
wherein the controller (51) **is** configured to count the energy delivered to the patient tissue based on at least one of a type of the at least one gas, a flow rate of the at least one gas and/or a power setting of the electrosurgical setting.

## Patentansprüche

1. Elektrochirurgischer Generator (50, 61), umfassend:
eine Leistungszufuhr (52), die elektrochirurgische Energie an einen Applikator (10) über eine Hochfrequenz(HF-)Ausgabestufe (54) zuführt;
einen Speicher (60), der zumindest eine Energiequantifizierungsfunktion speichert, die eine Energiemenge bestimmt, die an Patientengewebe durch den Applikator (10) abgegeben wird, wobei die zumindest eine Energiequantifizierungsfunktion tatsächliche Energie, die an eine Gewebeoberfläche abgegeben wird, mit der Ausgabeleistung der HF-Ausgabestufe (54) assoziiert; und
eine Steuerung (51), welche die Energiemenge, die an das Patientengewebe abgegeben wird, basierend auf der Energiequantifizierungsfunktion und der Ausgabeleistung der HF-Ausgabestufe (54) bestimmt.

2. Elektrochirurgischer Generator (50, 61) nach Anspruch 1, wobei die Steuerung dazu konfiguriert ist, die Ausgabeleistung basierend auf einer ausgewählten Generatorleistungseinstellung zu bestimmen.

3. Elektrochirurgischer Generator (50, 61) nach Anspruch 1, wobei die Steuerung dazu konfiguriert ist, die Ausgabeleistung basierend auf Abtastausgabespannung und -strom der HF-Ausgabestufe (54) zu bestimmen.

4. Elektrochirurgischer Generator (50, 61) nach Anspruch 1, ferner umfassend eine Eingabe-/Ausgabe-Schnittstelle (56), die dazu konfiguriert ist, die Energiemenge, die an das Patientengewebe abgegeben wird, anzuzeigen;
wobei die Energiemenge, die an das Patientengewebe abgegeben wird, in Joule angezeigt wird.

5. Elektrochirurgischer Generator (50, 61) nach Anspruch 2, wobei die Steuerung (51) dazu konfiguriert ist, die Energie, die an das Patientengewebe abgegeben wird, basierend auf der ausgewählten Leistungseinstellung und einer Dauer der Aktivierungszeit des Applikators (10) bei der ausgewählten Leistungseinstellung zu zählen.

6. Elektrochirurgischer Generator (50, 61) nach Anspruch 2, ferner umfassend zumindest einen Sensor (64), der an eine Ausgabe der HF-Ausgabestufe (54) gekoppelt ist, wobei der Sensor (64) dazu konfiguriert ist, Spannung und/oder Strom der HF-Ausgabestufe (54) abzutasten und die abgetastete Spannung und/oder den abgetasteten Strom an die Steuerung (51) bereitzustellen.

7. Elektrochirurgischer Generator (50, 61) nach Anspruch 5, ferner umfassend zumindest einen Sensor (64), der Impedanz an der HF-Ausgabestufe (54) misst und die gemessene Impedanz an die Steuerung (51) bereitstellt, wobei die Steuerung (51) basierend auf der gemessenen Impedanz bestimmt, ob der Applikator (10) Energie an das Patientengewebe oder an Umgebungsluft anlegt, und die Steuerung (51) die abgegebene Energie nur zu der Zählung hinzufügt, wenn der Applikator (10) Energie an das Patientengewebe anlegt.

8. Elektrochirurgischer Generator (50, 61) nach Anspruch 1, ferner umfassend eine Eingabe-/Ausgabe-Schnittstelle (56), die Auswahl eines Energieendpunktes für einen Vorgang ermöglicht, wobei die Steuerung (51) dazu konfiguriert ist, zu bewirken, dass die Leistungszufuhr (52) Zuführen von elektrochirurgischer Energie an den Applikator (10) stoppt, wenn die Zählung den Energieendpunkt überschreitet.

9. Elektrochirurgischer Generator (50, 61) nach Anspruch 8, wobei die Steuerung (51) dazu konfiguriert ist, eine Benachrichtigung über die Eingabe-/Ausgabe-Schnittstelle (56) auszulösen, wenn die Zählung den Energieendpunkt überschreitet.

10. Elektrochirurgischer Generator (50, 61) nach Anspruch 8, wobei der Speicher (60) einen vorbestimmten Energieendpunkt für jeden aus einer Vielzahl von Vorgängen speichert;
wobei die Eingabe-/Ausgabe-Schnittstelle (56) Auswahl von zumindest einem aus der Vielzahl von Vorgängen ermöglicht, wobei bei Auswahl von zumindest einem Vorgang die Steuerung (51) einen entsprechenden Energieendpunkt aus dem Speicher (60) abruft.

11. Elektrochirurgischer Generator (50, 61) nach Anspruch 1, wobei die Steuerung dazu konfiguriert ist, die zumindest eine Energiequantifizierungsfunktion basierend auf einem Typ von Applikator (10) auszuwählen.

12. Elektrochirurgischer Generator (50, 61) nach Anspruch 1, wobei der Applikator (10) und zumindest eine Aufnahme (30, 32, 34, 36, 40) konfiguriert sind, sodass zumindest eine Energiequantifizierungsfunktion von dem Applikator (10) beim Koppeln des Applikators (10) an zumindest eine Aufnahme (30, 32, 34, 36, 40) empfangen wird.

13. Elektrochirurgischer Generator (50, 61) nach Anspruch 5, ferner umfassend eine Eingabe-/Ausgabe-Schnittstelle (56), die Speichern einer Gesamtenergiezählung, die an einen ersten Behandlungsbereich eines Patienten abgegeben wird, als einen Energieendpunkt in einem Speicher ermöglicht, wobei bei Auswahl eines Vorgangs für einen kontralateralen Behandlungsbereich des Patienten die Steuerung (51) einen gespeicherten Energieendpunkt aus dem Speicher (60) abruft.

14. Elektrochirurgischer Generator (50, 61) nach Anspruch 1, wobei bei Abschluss eines Vorgangs an einen ersten Behandlungsbereich eines Patienten die Steuerung (51) eine Gesamtenergiemenge, die an das Patientengewebe abgegeben wird, bestimmt und die bestimmte Gesamtenergiemenge in dem Speicher (60) als einen Energieendpunkt für einen Vorgang an einen kontralateralen Behandlungsbereich des Patienten speichert;
wobei der elektrochirurgische Generator (50, 61) ferner eine Eingabe-/Ausgabe-Schnittstelle (56) umfasst, die Auswahl eines Vorgangs für den kontralateralen Behandlungsbereich ermöglicht, wobei bei Auswahl des Vorgangs die Steuerung (51) den gespeicherten Energieendpunkt aus dem Speicher (60) abruft.

15. Elektrochirurgischer Generator (50, 61) nach Anspruch 1, ferner umfassend eine Flusssteuerung (62), die dazu konfiguriert ist, zumindest ein Gas an den Applikator (10) bereitzustellen, wobei der Applikator (10) dazu konfiguriert ist, Plasma aus der elektrochirurgischen Energie und dem zumindest einen Gas zu erzeugen, wobei das Plasma an das Patientengewebe abzugeben ist;
wobei die Steuerung (51) dazu konfiguriert ist, die Energie, die an das Patientengewebe abgegeben wird, basierend auf zumindest einem von einem Typ des zumindest einen Gases, einer Flussrate des zumindest einen Gases und/oder einer Leistungseinstellung der elektrochirurgischen Einstellung zu zählen.

## Revendications

1. Générateur électro-chirurgical (50, 61) comprenant :
un bloc d'alimentation (52) qui fournit de l'énergie électro-chirurgicale à un applicateur (10) via un étage de sortie radio-fréquence (RF) (54) ;
une mémoire (60) qui stocke au moins une fonction de quantification d'énergie qui détermine une quantité d'énergie administrée au tissu du patient par l'applicateur (10), l'au moins une fonction de quantification d'énergie associe l'énergie effective administrée à une surface de tissu à la puissance de sortie de l'étage de sortie RF (54) ; et
un contrôleur (51) qui détermine la quantité d'énergie administrée au tissu du patient sur la base de la fonction de quantification d'énergie et de la puissance de sortie de l'étage de sortie RF (54).

2. Générateur électro-chirurgical (50, 61) selon la revendication 1, dans lequel le contrôleur est configuré pour déterminer la puissance de sortie sur la base d'un réglage de puissance de générateur sélectionné.

3. Générateur électro-chirurgical (50, 61) selon la revendication 1, dans lequel le contrôleur est configuré pour déterminer la puissance de sortie sur la base de la tension et du courant de sortie d'échantillonnage de l'étage de sortie RF (54).

4. Générateur électro-chirurgical (50, 61) selon la revendication 1, comprenant en outre une interface d'entrée/sortie (56) configurée pour afficher la quantité d'énergie administrée au tissu du patient ;
dans lequel la quantité d'énergie administrée au tissu du patient est affichée en Joules.

5. Générateur électro-chirurgical (50, 61) selon la revendication 2, dans lequel le contrôleur (51) est configuré pour compter l'énergie administrée au tissu du patient sur la base du réglage de puissance sélectionné et d'une durée de temps d'activation de l'applicateur (10) au réglage de puissance sélectionné.

6. Générateur électro-chirurgical (50, 61) selon la revendication 2, comprenant en outre au moins un capteur (64) couplé à une sortie de l'étage de sortie RF (54), le capteur (64) étant configuré pour échantillonner la tension et/ou le courant de l'étage de sortie RF (54) et fournir la tension et/ou le courant échantillonné au contrôleur (51).

7. Générateur électro-chirurgical (50, 61) selon la revendication 5, comprenant en outre au moins un capteur (64) qui mesure l'impédance à l'étage de sortie RF (54) et fournit l'impédance mesurée au contrôleur (51), dans lequel le contrôleur (51) détermine si l'applicateur (10) applique l'énergie au tissu du patient ou à l'air ambiant sur la base de l'impédance mesurée et le contrôleur (51) ajoute l'énergie administrée au compte uniquement lorsque l'applicateur (10) applique l'énergie au tissu du patient.

8. Générateur électro-chirurgical (50, 61) selon la revendication 1, comprenant en outre une interface d'entrée/sortie (56) qui permet la sélection d'un seuil d'énergie pour une procédure, dans lequel le contrôleur (51) est configuré pour amener le bloc d'alimentation (52) à cesser de fournir l'énergie électro-chirurgicale à l'applicateur (10) lorsque le compte dépasse le seuil d'énergie.

9. Générateur électro-chirurgical (50, 61) selon la revendication 8, dans lequel le contrôleur (51) est configuré pour déclencher une notification via l'interface d'entrée/sortie (56) lorsque le compte dépasse le seuil d'énergie.

10. Générateur électro-chirurgical (50, 61) selon la revendication 8, dans lequel la mémoire (60) stocke un seuil d'énergie prédéterminé pour chacune d'une pluralité de procédures ;
dans lequel l'interface d'entrée/sortie (56) permet la sélection d'au moins l'une de la pluralité de procédures, dans lequel lors de la sélection d'au moins une procédure, le contrôleur (51) récupère un seuil d'énergie correspondant à partir de la mémoire (60).

11. Générateur électro-chirurgical (50, 61) selon la revendication 1, dans lequel le contrôleur est configuré pour sélectionner l'au moins une fonction de quantification d'énergie sur la base d'un type d'applicateur (10).

12. Générateur électro-chirurgical (50, 61) selon la revendication 1, dans lequel l'applicateur (10) et au moins un réceptacle (30, 32, 34, 36, 40) sont configurés de sorte qu'au moins une fonction de quantification d'énergie soit reçue de l'applicateur (10) lors du couplage de l'applicateur (10) à au moins un réceptacle (30, 32, 34, 36, 40).

13. Générateur électro-chirurgical (50, 61) selon la revendication 5, comprenant en outre une interface d'entrée/sortie (56) qui permet de stocker en mémoire un compte total d'énergie administrée à une première zone de traitement d'un patient en tant que seuil d'énergie, dans lequel lors de la sélection d'une procédure pour une zone de traitement controlatérale du patient, le contrôleur (51) récupère un seuil d'énergie stocké à partir de la mémoire (60).

14. Générateur électro-chirurgical (50, 61) selon la revendication 1, dans lequel à l'achèvement d'une procédure sur une première zone de traitement d'un patient, le contrôleur (51) détermine une quantité totale d'énergie administrée au tissu du patient et stocke la quantité totale d'énergie déterminée dans la mémoire (60) en tant que seuil d'énergie pour une procédure sur une zone de traitement controlatérale du patient ;
le générateur électro-chirurgical (50, 61) comprenant en outre une interface d'entrée/sortie (56) qui permet la sélection d'une procédure pour la zone de traitement controlatéral, dans lequel lors de la sélection de la procédure, le contrôleur (51) récupère le seuil d'énergie stockée à partir de la mémoire (60).

15. Générateur électro-chirurgical (50, 61) selon la revendication 1, comprenant en outre un contrôleur de flux (62) configuré pour fournir au moins un gaz à l'applicateur (10), dans lequel l'applicateur (10) est configuré pour générer du plasma à partir de l'énergie électro-chirurgicale et de l'au moins un gaz, le plasma devant être administré au tissu du patient ;
dans lequel le contrôleur (51) est configuré pour compter l'énergie administrée au tissu du patient sur la base d'au moins l'un d'un type de l'au moins un gaz, d'un débit de l'au moins un gaz et/ou d'un réglage de puissance du réglage électro-chirurgical.
